# FASCICULE DE BREVET EUROPEEN

(11) **EP 0 516 567 B1**
(45) Date de publication et mention de la délivrance du brevet: **16.07.1997**
(21) Numéro de dépôt: 92420179.1
(22) Date de dépôt: 27.05.1992
(51) Int. Cl.: A61B 17/58

(54) **Dispositif de stabilisation intervertébrale à amortisseurs**
Stossdämpfende Vorrichtung zur Zwischenwirbelstabilisierung
Shock-absorbing device for intervertebral stabilisation

(30) Priorité: 30.05.1991 FR 9106695
(43) Date de publication de la demande: 02.12.1992
(73) Titulaire: Société dite: "PSI", 69260 Charbonnières-les-Bains (FR)
(72) Inventeur: Navas, Fernand, F-69260 Charbonnières-les-Bains (FR)
(74) Mandataire: Karmin, Roger

(56) Documents cités:
- EP-A- 0 322 334
- EP-A- 0 346 269
- EP-A- 0 381 588
- EP-A- 0 478 470
- GB-A- 2 168 255
- JOURNAL OF BONE AND JOINT SURGERY. vol. 63-A, no. 8, Octobre 1981, BOSTON US pages 1289 - 1291; M.PRITSCH ET AL.: 'Manipulation and External Fixation of Metacarpal Fractures'

## Description

La présente invention se réfère aux dispositifs de stabilisation intervertébrale destinés au maintien en position relative convenable d'au moins deux vertèbres dont le disque commun est dégradé.

On sait qu'au cours du vieillissement, les disques inter-vertébraux risquent de se dégrader, de telle sorte que les mouvements de l'articulation intervertébrale se modifient, si bien qu'ils deviennent anormalement plus amples. Les vertèbres peuvent alors se déplacer de manière excessive les unes par rapport aux autres, jusqu'à entraîner des déplacements permanents du fait que les vertèbres se positionnent mal.

Le disque intervertébral se comporte plus comme un répartiteur de pression ou un coupleur tridimensionnel de mouvements que comme un simple amortisseur de charges longitudinales.

Le mouvement intervertébral est guidé par le jeu des articulaires postérieures. Ces dernières n'ont qu'un seul degré de liberté ; les deux facettes ne pouvant que glisser l'une sur l'autre.

Lors de ce mouvement, le disque se laisse déformer d'une façon élastique en freinant progressivement le mouvement pour l'amortir complètement en fin d'amplitude.

Le disque possède une qualité visco-élastique. Il s'adapte progressivement par viscosité à un nouveau rapport anatomique. De ce fait, le mouvement de retour est alors pris en charge par un amortisseur progressif similaire et ceci rapidement dès son origine.

Le jeu des facettes articulaires peut être asymétrique, et de ce fait, créer un mouvement tridimensionnel couplant flexion latérale et rotation horizontale.

Ce mouvement complexe est lui-même couplé au grand mouvement de flexion-extension. En flexion complète comme en extension complète l'amplitude du mouvement combiné (flexion-rotation) devient nulle alors qu'il obtient un maximum d'amplitude en position anatomique, c'est-à-dire dans le rapport anatomique naturel des vertèbres lorsque l'on se tient debout ou lorsque l'on marche (en fait dans une position intermédiaire entre flexion et extension maximale).

La dégradation biologique du disque vient perturber cette mécanique de mouvements couplés et amortis.

Cette évolution est source d'inconfort et de douleur.

On a déjà proposé, par exemple dans le brevet américain 4 743 260, de placer entre au moins deux vertèbres voisines un dispositif de stabilisation flexible réalisé au moyen de deux éléments fixés aux vertèbres considérées. Les éléments de stabilisation sont réalisés en une matière non métallique résistante mais suffisamment flexible pour permettre au moins un mouvement normal de la colonne vertébrale.

Les éléments de stabilisation considérés sont prévus en une matière plastique renforcée de fibres de carbone, de telle sorte qu'ils comportent une certaine flexibilité. Toutefois, cette dernière est limitée à de très faibles amplitudes dans le sens de leurs courbures, mais ils ne comportent aucune élasticité en traction ou en compression.

On a ainsi proposé de placer un lien souple, non élastique, entre deux vertèbres, et qui limite comme un frein brutal le mouvement dans son amplitude en flexion. A ce stade, il se comporte comme un système rigide, reportant les contraintes mécaniques sur les articulations intervertébrales adjacentes.

Ceci ramène aux complications de surcharge mécanique comme pour les arthrodèses ou les montages métalliques rigides.

D'autre part, le mouvement pathologique dégénératif entre deux vertèbres n'est pas seulement un mouvement d'amplitude exagérée mais aussi une subtile désorganisation du couplage tridimensionnel de différents degrés de liberté.

Un simple lien souple ne peut aider que dans la mesure où il positionne les deux vertèbres dans une amplitude extrême et donc en extension.

Les micro-mouvements résiduels alors possibles grâce au fluage du lien sont susceptibles d'apporter une adaptation élémentaire et grossière par rapport aux besoins fonctionnels. Mais leur existence différencie nettement cette fixation par rapport au système rigide (arthrodèse ou fixation métallique)

On connaît également d'après le document EP 0 346 269 une endoprothèse pour disque vertébral, comprenant un tube ondulé circulaire ou elliptique entourant un matériau visco-élastique, et des plaques de recouvrement permettant la fixation et l'orientation dudit tube. Cette endoprothèse est prévue pour être placée entre deux vertèbres après resection totale du disque intervertébral pour constituer un logement suffisant en vue de la mise en place de l'endoprothèse.

La structure de l'endoprothèse du brevet EP 0 346 269 va à l'encontre d'un dispositif permettant de supporter des efforts de traction et de compression soumis aux vertèbres d'une colonne vertébrale. En effet, le tube ondulé est réalisé en titane, empêchant le retour en position initiale de l'endoprothèse après un allongement total. Le tube ondulé soumis à un effort de traction risque de se déformer complètement et d'agir comme une cale rigide risquant d'endommager sérieusement les vertèbres du patient.

En outre, on remarque que le tube ondulé ne peut se déplacer que parallèlement à l'axe de l'endoprothèse. Ce déplacement est totalement contraire au mouvement soumis aux vertèbres d'une colonne vertébrale.

Les perfectionnements qui font l'objet de la présente invention visent à permettre la réalisation de dispositifs susceptibles d'accompagner le vieillissement des disques et qui constituent donc des prothèses évitant les inconvénients mentionnés en tête des présentes.

On comprend que le but du système proposé est de suppléer le plus physiologiquement possible aux insufffisances du disque créées par les conditions biologiques et pathologiques.

Le système proposé vise à obtenir une nouvelle stabilité à partir d'une position intervertébrale se rapprochant de la position neutre (entre flexion-extension).

Dans cette position, la possibilité de mouvements des facettes articulaires postérieures est plus grand en particulier dans le sens de l'extension.

D'autre part, le travail asymétrique des deux facettes postérieures est possible.

La stabilité de l'articulation intervertébrale est alors obtenue grâce à la qualité d'amortisseur du dispositif suivant l'invention.

Ce dernier a pour rôle d'accompagner le mouvement de l'articulation en le limitant légèrement en flexion et en évitant les déplacements anormaux. Parallèlement, tout en maintenant les vertèbres en extension l'une par rapport à l'autre, il évite un trop grand appui des facettes articulaires l'une sur l'autre. Le système suivant l'invention limite également la fermeture du recessus latéral en empêchant de ce fait la compression éventuelle de la racine nerveuse.

A cet effet, le dispositif suivant l'invention comprend au moins un amortisseur propre à résister élastiquement d'une part à un allongement et d'autre part à une compression axiale, tandis que l'amortisseur affecte la forme d'un corps allongé pourvu d'une partie centrale reliée à deux extrémités renflées qui coopèrent respectivement avec un implant ancré dans la vertèbre correspondante.

Ainsi, l'amortisseur suivant l'invention peut exercer une force distractante ou compressive, ce qui lui permet d'agir de façon permanente sur la mauvaise position intervertébrale.

Autrement dit, le dispositif de stabilisation suivant l'invention guide et limite le mouvement de l'articulation intervertébrale, tout en étant susceptible d'exercer des forces permanentes modifiant la position des vertèbres entre elles.

Le dispositif de stabilisation suivant l'invention est capable d'amortir le mouvement en flexion comme celui en extension et de ce fait d'autoriser un travail asymétrique des facettes des vertèbres tout en autorisant le mouvement couplé tridimensionnel qui en naît.

On se rapproche ainsi d'un mouvement physiologique complexe et amorti.

Bien entendu, on peut utiliser un seul dispositif suivant l'invention ou plusieurs pour relier deux vertèbres voisines.

Le dessin annexé, donné à titre d'exemple, pemettra de mieux comprendre l'invention, les caractéristiques qu'elle présente et les avantages qu'elle est susceptible de procurer:
Figure 1 est une vue en perspective des différents éléments constituant un dispositif de stabilisation suivant l'invention;
Figure 2 est une vue par derrière de trois vertèbres associées à des dispositifs de stabilisation suivant l'invention.
Figure 3 est une coupe suivant III-III (fig. 2).
Figure 4 est une vue en perspective de deux manchons destinés à coopérer avec les cols de l'amortisseur d'un dispositif conforme à l'invention.
Fig. 5 est une vue semblable à celle de fig. 3, mais illustrant le montage d'un dispositif suivant l'invention en position antérieure.
Fig. 6 est une vue en coupe très schématique du principe d'un amortisseur réalisé suivant une variante de l'invention.
Fig. 7 montre en coupe à l'état libre les deux éléments de l'amortisseur de fig. 1.
Fig. 8, 9 et 10, sont des coupes longitudinales de deux modes différents de réalisation pratique de l'amortisseur illustré en fig. 6.

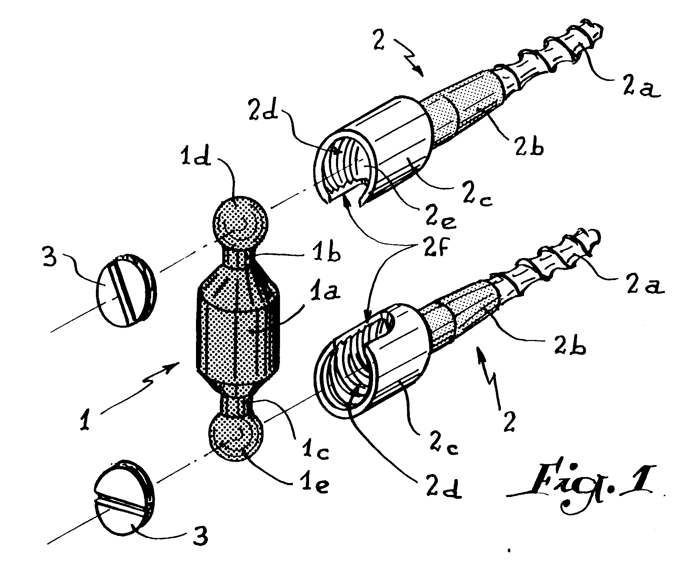

Le dispositif suivant l'invention, qui a été illustré en fig. 1, comprend essentiellement un amortisseur 1 réalisé en une matière bio-compatible et élastique et deux implants 2 vissés dans deux vertèbres voisines et dont les extrémités libres sont associées aux deux extrémités de l'amortisseur 1.

On observe que l'amortisseur 1 est réalisé sous la forme d'un corps allongé pourvu d'une partie centrale ventrue 1a reliée par deux cols 1b, 1c à deux extrémités renflées 1d, 1e. Dans un mode d'exécution avantageux de la disposition qui précède, la partie ventrue 1a peut être prévue de section longitudinale elliptique, tandis que les deux extrémités 1d et 1c affectent chacune la forme d'une sphère. Bien entendu, la forme de la partie ventrue 1a peut être de section cylindrique avec deux embouts tronconiques être réalisée sous la forme de deux troncs de cône ou encore être asymétrique dans des applications particulières.

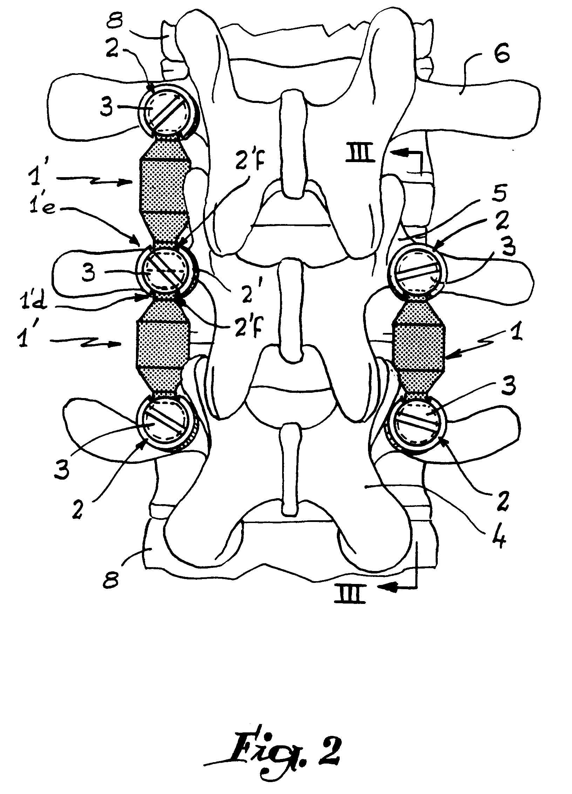

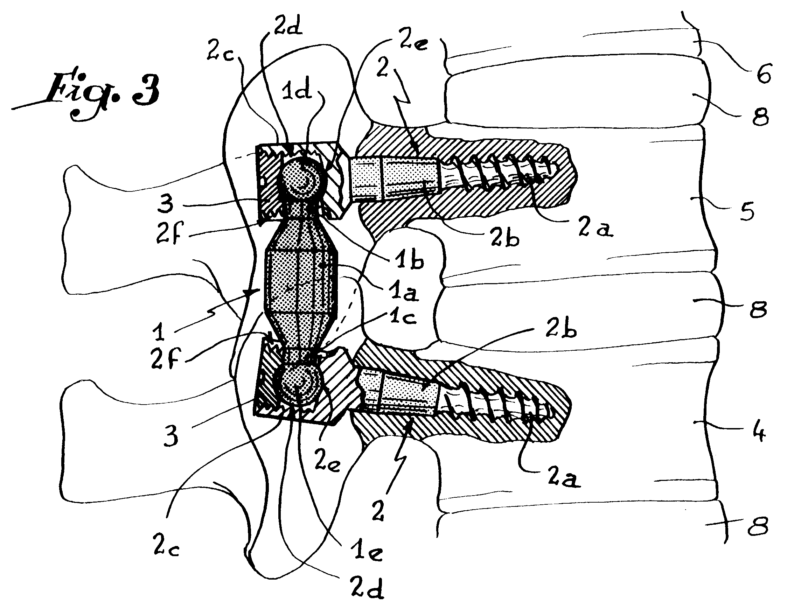

Chaque implant 1 comporte tout d'abord une vis 2a propre à se visser dans le pédicule d'une vertèbre ou dans tout autre endroit de celle-ci. La vis 2a se prolonge par un corps cylindrique 2b qui se termine par une cuvette 2c de forme cylindrique creuse pourvue d'une paroi intérieure taraudée 2d et d'un fond 2e concave présentant une forme complémentaire à celle de la moitié de l'extrémité 1d, 1e de l'amortisseur. On observe que la cuvette 2c est pourvue d'une encoche latérale 2f destinée à permettre le passage du col 1b, 1c de l'amortisseur 1 pour la mise en place de celui-ci par rapport aux implants. Le verrouillage des extrémités de l'amortisseur 1 s'effectue après leur mise en place dans les cuvettes 2c en vissant un embout fileté 3 à l'intérieur de la cuvette correspondante par rapport à la paroi taraudée 2d. Bien entendu, la base 3a de l'embout 3 est prévue concave et hémisphérique, de manière à coopérer exactement avec les extrémités sphériques 1d, 1e de l'amortisseur. On a illustré en fig. 2 et 3 le montage d'un dispositif suivant l'invention par rapport à deux vertèbres voisines 4 et 5 d'un rachis.

Sur la droite de fig. 2, on a illustré un dispositif comportant un seul amortisseur 1 associé à deux implants 2 assujettis chacun à une vertèbre 4, 5. On peut prévoir le même montage sur la partie gauche. En outre, il est possible que trois vertèbres successives 4, 5, 6 aient besoin d'une stabilisation. Dans ce cas, l'un des implants 2' comporte deux encoches diamétralement opposées 2'f, tandis que les deux amortisseurs 1' comportent chacun une extrémité 1'd, 1'e, tronquée suivant un plan diamétral de la sphère perpendiculaire à l'axe longitudinal de l'amortisseur afin que les deux extrémités tronquées 1'd, 1'e puissent être retenues dans la cuvette de l'implant 2' (voir la partie gauche de fig. 2).

Fig. 3 illustre de manière très détaillée la structure de l'assemblage des extrémités de l'amortisseur avec deux implants. On retrouve la cuvette creuse 2c à fond concave bombé 2e ainsi que l'embout 3 à base bombée concave 3a afin que les deux extrémités sphériques 1d, 1e de l'amortisseur 1 soient convenablement verrouillées par rapport aux implants 2. Ce verrouillage permet de créer une sorte d'articulation à rotule facilitant les mouvements du rachis.

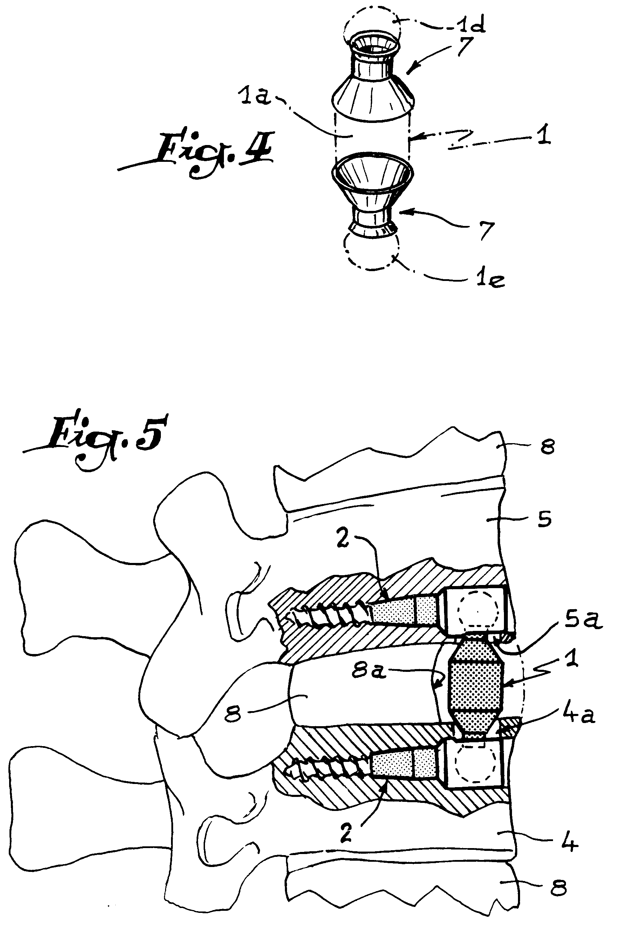

Comme illustré en fig. 4, les cols 1b, 1c de l'amortisseur 1 sont avantageusement protégés par un manchon 7 réalisé en un métal ou en une toute autre matière rigide et qui assure la qualité mécanique de la relation entre l'amortisseur et les implants. Les manchons 7 peuvent comprendre sur leurs faces intérieures des crans qui interviennent de manière active en diminuant des efforts mécaniques au niveau du col correspondant.

Comme illustré en fig. 2 et 3, le dispositif de stabilisation suivant l'invention est positionné soit sur la face postérieure, soit sur celle latérale des vertèbres. Il peut être aussi utilisé en avant sur le corps vertébral, tel qu'illustré en fig. 5.

Dans ce mode de mise en place, il va de soi que les implants 2 devront être disposés latéralement en dehors des vaisseaux ou le dispositif sera posé comme illustré en fig. 5, c'est-à-dire noyé dans les vertèbres.

Dans ce cas, on effectue une légère résection du disque intervertébral 8 pour constituer une dépression 8a dans celui-ci. Les implants 2 sont enfoncés profondément dans la vertèbre, de manière que leur cuvette 2c soit noyée dans la vertèbre qui est elle-même entaillée en 4a, 5a afin de permettre le passage des deux cols de l'amortisseur. On est ainsi assuré que le dispositif n'interfère pas avec les vaisseaux disposés le long de la face antérieure du rachis.

Les amortisseurs peuvent être prévus de différentes longueurs variant de quelques millimètres les unes par rapport aux autres, de manière que l'on puisse ajuster la longueur de l'amortisseur à la pathologie du patient.

On a ainsi réalisé un système de stabilisation permettant d'obtenir un jeu des mouvements résiduels intervertébraux nécessaires à la physiologie élémentaire du rachis, tout en éliminant les mauvaises positions des vertèbres et leurs mouvements anormaux.

Toute matière appropriée peut être adoptée pour la réalisation de l'amortisseur 1, en particulier un élastomère bio-compatible. On pourrait encore adopter une matière composite répondant au mieux aux deux exigences mécaniques de l'amortisseur, soit la résistance à une traction longitudinale et à une compression sans flambage. Les matériaux choisis peuvent être de la même famille ou totalement différents.

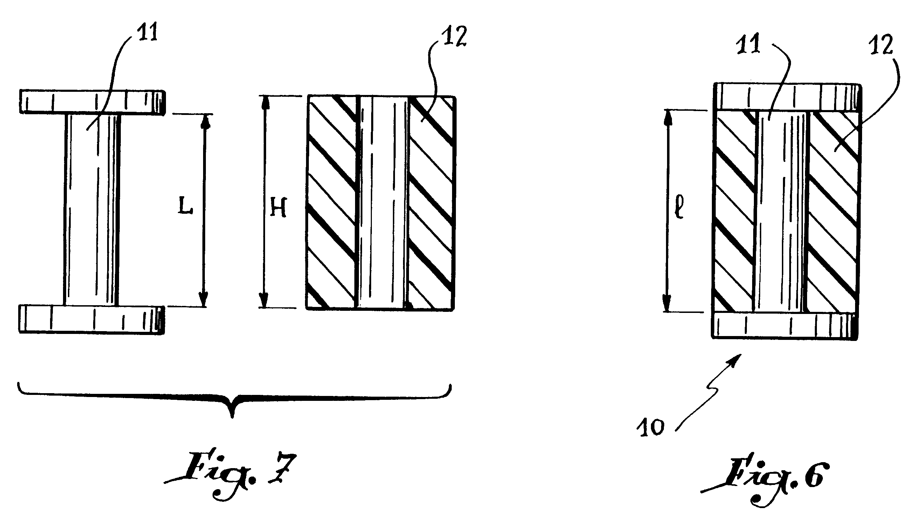

Le principe de l'amortisseur réalisé en une matière composite est illustré en fig. 6 et 7. L'amortisseur référencé 10 comprend deux éléments 11 et 12 réalisés tous les deux en des matières élastiques bio-compatibles. Le premier élément 11 est réalisé sous la forme d'une bobine dont la distance entre les joues est référencée L à l'état libre. Le second élément référencé 12 affecte la forme d'un manchon tubulaire de hauteur H à l'état libre. Le montage de l'amortisseur consiste à placer l'élément 12 entre les joues de l'élément 11 après que celui-ci ait été allongé. Donc, l'élément 11 comprime par ses joues l'élément 12 dans le sens de la compression, tandis que ce dernier maintient l'élément 11 dans une position prétendue. Ainsi, la longueue l du manchon après montage et qui correspond à la distance entre les joues de l'élément 12 se définit par la relation L < l < H.

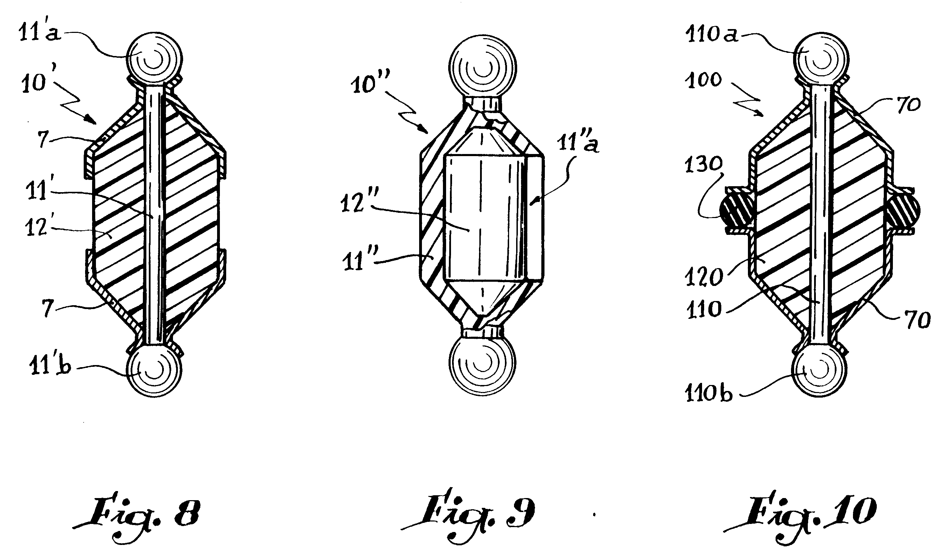

Dans une première réalisation pratique illustrée en fig. 8, l'élément 11' ou âme affecte la forme d'une haltère, tandis que l'élément 12 est réalisé sous la forme d'un corps 12' dont la forme générale est celle du centre de l'amortisseur 1 de fig. 1, 2 et 3.

Pour réaliser un tel amortisseur, on fabrique tout d'abord l'âme 11'. La tige de celle-ci est allongée élastiquement dans le sens axial, puis le corps 12' est surmoulé sur cette âme. Après la fabrication, l'âme 11'a est pré-tendue, tandis que le corps 12' est précomprimé. On note que la liaison entre l'âme 11' et le corps 12' s'effectue au moyen des deux manchons 7, les extrémités de l'âme 11' dépassant au delà des bouts du corps 12', tandis que les deux têtes sphériques 11'a, 11'b de ladite âme correspondent aux boules 1d, 1e de l'amortisseur 1.

Dans un second mode d'exécution illustré en fig. 9, l'amortisseur référencé 10'' comprend une enveloppe élastique 11'' dont la forme extérieure correspond à celle de l'amortisseur 1, ainsi qu'un bloc de matière élastique 12''. Après fabrication, l'enveloppe 11'' qui comporte une cavité intérieure creuse, est allongée de manière à augmenter la hauteur de sa cavité dans laquelle le bloc 12'' est introduit par une ouverture latérale 11''a ou autrement. L'enveloppe 11'' est en conséquence pré-tendue dans le sens de l'allongement, tandis que le bloc 12'' est pré-comprimé par l'action de l'enveloppe.

Dans une dernière réalisation pratique illustrée en fig. 10, l'amortisseur 100 comprend un corps 120 qui est monté sur un élément 110 en forme d'haltère comme déjà représenté en fig. 8. La liaison entre l'élément 110 et le corps 120 s'effectue au moyen de deux manchons 70 dont l'une de leurs extrémités est en appui contre les têtes sphériques 110a et 110b de l'élément 110 correspondant aux boules 1d, 1e de l'amortisseur 1. Les autres extrémités des manchons 70 sont respectivement recourbées en direction de l'extérieur de l'amortisseur 100 afin de former un espace libre circulaire à l'intérieur duquel est introduit un joint torique 130. Ce dernier permet de compenser les efforts de compression de l'amortisseur 100.

On constate que le joint torique 130 peut être réalisé en une matière bio-compatible semblable à celle du corps 120 de manière à pouvoir résister aux efforts de compression. Le joint 130 et l'élément 110 en forme d'haltère peuvent être utilisés ensemble sur le même amortisseur ou indépendamment l'un de l'autre suivant les contraintes à réguler.

La dureté des éléments composants l'amortisseur 10', 10'' sera choisie de manière que sous l'effet des forces appliquées par les vertèbres, aucun des éléments ne reprenne ses dimensions à l'état libre.

Ainsi, que l'amortisseur soit soumis à une force de traction ou à une force de compression, il demeure toujours pré-contraint, si bien que la force qui lui est appliquée est toujours amortie à quel qu'endroit que l'on se situe dans l'amplitude du mouvement imposé à l'amortisseur composite.

## Revendications

1. Dispositif de stabilisation intervertébrale constitué par un élément flexible mis en place entre au moins deux vertèbres par l'intermédiaire de deux implants respectivement associés à chaque vertèbre, caractérisé en ce qu'il comprend au moins un amortisseur (1; 10; 10'; 10''; 100) propre à résister élastiquement d'une part à un allongement et d'autre part à une compression axiale, tandis que l'amortisseur (1; 10; 10'; 10''; 100) affecte la forme d'un corps allongé pourvu d'une partie centrale (1a; 12; 12'; 12''; 120) reliée à deux extrémités renflées (1d, 1e ; 11'a, 11'b ; 110a, 110b) qui coopèrent respectivement avec un implant (2) ancré dans la vertèbre (4, 5) correspondante.

2. Dispositif suivant la revendication 1, caractérisé en ce que l'amortisseur (1; 10; 10'; 10''; 100) comporte des manchons rigides (7 ; 70) qui sont placés au voisinage des extrémités (1d, 1e ; 11'a, 11'b ; 110a, 110b).

3. Dispositif suivant la revendication 1, caractérisé en ce que l'amortisseur (1, 10, 10', 10'', 100) est réalisé en une matière élastique bio-compatible telle qu'un élastomère.

4. Dispositif suivant la revendication 1, caractérisé en ce que l'amortisseur (1, 10, 10', 10'', 100) est réalisé en une matière bio-compatible à deux composants.

5. Dispositif suivant la revendication 1, caractérisé en ce que la partie centrale (1a; 12; 12'; 12''; 120) de l'amortisseur (1; 10; 10'; 10''; 100) présente en section longitudinale une forme elliptique.

6. Dispositif suivant la revendication 1, caractérisé en ce que la partie centrale (1a; 12; 12'; 12''; 120) de l'amortisseur (1; 10; 10'; 10''; 100) est asymétrique.

7. Dispositif suivant la revendication 1, caractérisé en ce que les implants (2) qui recoivent les extrémités renflées (1d, 1e; 11'a, 11'b; 110a, 110b) de l'amortisseur (1; 10; 10'; 10''; 100) sont chacun réalisés sous la forme d'une cuvette (2c) dont le fond (2e) comporte un profil concave de forme appropriée à sa coopération étroite avec l'extrémité correspondante de l'amortisseur (1; 10; 10'; 10''; 100), ladite cuvette comportant des moyens (2d) de fixation d'un embout (3) destiné à appliquer ladite extrémité contre le fond (2e) et la cuvette (2c) de l'implant (2).

8. Dispositif suivant la revendication 7, caractérisé en ce que la cuvette (2c) de chaque implant (2) comporte un trou taraudé (2d) dont le fond (2e) est hémisphérique, cette cuvette étant munie d'une encoche (2f) afin qu'elle débouche latéralement vers l'extérieur, la fixation de l'extrémité de l'amortisseur s'effectuant au moyen d'un embout fileté (3) à base (3a) hémisphérique qui se visse dans la cuvette (2c).

9. Dispositif suivant la revendication 8, caractérisé en ce qu'il comprend des implants médiants (2') comportant deux encoches (2'f) se faisant face pour le maintien des extrémités de deux amortisseurs adjacents (1') dont au moins l'une des extrémités (1'd, 1'e) est une demi-sphère.

10. Dispositif suivant la revendication 4, caractérisé en ce que l'un des deux éléments de l'amortisseur (10, 10', 10'', 100) est pré-contraint dans le sens de l'extension et le second élément dans le sens de la compression.

11. Dispositif suivant la revendication 1, caractérisé en ce que l'amortisseur (10') comprend une âme élastique (11') réalisée sous la forme d'une haltère dont la tige est élastiquement allongée ainsi qu'un corps (12') pré-contraint dans le sens de la compression par l'élasticité de l'âme (11').

12. Dispositif suivant la revendication 1, caractérisé en ce que l'amortisseur (10'') est réalisé sous la forme d'une enveloppe élastique (11'') pré-tendue élastiquement dans le sens axial en vue de l'allonger et qui comporte une cavité renfermant un bloc de matière élastique (12'') que l'enveloppe (11''), pré-contraint par compression.

13. Dispositif suivant la revendication 1, caractérisé en ce que l'amortisseur (100) comprend un élément (110) et un corps (120) reliés par deux manchons (70) dont l'une de leurs extrémités est recourbée en direction de l'extérieur pour former un espace libre circulaire à l'intérieur duquel est introduit un joint torique (130).

14. Dispositif suivant la revendication 13, caractérisé en ce que le joint torique (130) est réalisé en une matière bio-compatible suffisamment résistante pour pouvoir compenser les efforts de compression.

## Claims

1. An intervertebral stabilisation device formed by a flexible element placed between at least two vertebrae via two implants respectively associated with each vertebra, characterised in that it comprises at least one damper (1; 10; 10'; 10''; 100) suitable for elastically resisting firstly elongation and secondly axial compression, whilst the damper (1; 10; 10'; 10''; 100) assumes the form of an elongate body provided with a central part (1a; 12; 12'; 12''; 120) connected to two bulging ends (1d, 1e; 11'a, 11'b; 110a, 110b) which cooperate respectively with an implant (2) anchored in the corresponding vertebra (4, 5).

2. A device according to Claim 1, characterised in that the damper (1; 10; 10'; 10''; 100) comprises rigid sleeves (7; 70) which are placed in the vicinity of the ends (1d, 1e; 11'a, 11'b; 110a, 110b).

3. A device according to Claim 1, characterised in that the damper (1, 10, 10', 10'', 100) is made of a biocompatible elastic material such as an elastomer.

4. A device according to Claim 1, characterised in that the damper (1, 10, 10', 10'', 100) is made of a dual-component biocompatible material.

5. A device according to Claim 1, characterised in that the central part (1a; 12; 12'; 12''; 120) of the damper (1; 10; 10'; 10''; 100) is of elliptical shape in longitudinal section.

6. A device according to Claim 1, characterised in that the central part (1a; 12; 12'; 12''; 120) of the damper (1; 10; 10'; 10''; 100) is asymmetrical.

7. A device according to Claim 1, characterised in that the implants (2) which receive the bulging ends (1d, 1e; 11'a, 11'b; 110a, 110b) of the damper (1; 10; 10'; 10''; 100) are each made in the form of a cup (2c), the base (2e) of which comprises a concave profile of a shape appropriate to the close cooperation thereof with the corresponding end of the damper (1; 10; 10'; 10''; 100), said cup comprising means (2d) for fixing an end piece (3) intended to apply said end against the base (2e) and the cup (2c) of the implant (2).

8. A device according to Claim 7, characterised in that the cup (2c) of each implant (2) comprises a threaded hole (2d), the base (2e) of which is hemispherical, this cup being provided with a notch (2f) so that it opens laterally towards the outside, the fixing of the end of the damper being effected by means of a threaded end piece (3) having a hemispherical base (3a) which screws into the cup (2c).

9. A device according to Claim 8, characterised in that it comprises mediant implants (2') comprising two notches (2'f) facing one another for holding the ends of two adjacent dampers (1'), at least one of the ends (1'd, 1'e) of which is a hemisphere.

10. A device according to Claim 4, characterised in that one of the two elements of the damper (10, 10', 10'', 100) is prestressed in the direction of extension and the second element in the direction of compression.

11. A device according to Claim 1, characterised in that the damper (10') comprises an elastic core (11') made in the form of a dumbbell, the shank of which is elastically elongated, and a body (12') which is prestressed in the direction of compression by the elasticity of the core (11').

12. A device according to Claim 1, characterised in that the damper (10'') is made in the form of an elastic sheath (11'') pre-stretched elastically in the axial direction in order to elongate it and which comprises a cavity enclosing a block of elastic material (12'') pre-stressed in compression by the sheath (11'').

13. A device according to Claim 1, characterised in that the damper (100) comprises an element (110) and a body (120) connected by two sleeves (70), one of the ends of which is curved towards the outside to form a free circular space into which an O-ring (130) is introduced.

14. A device according to Claim 13, characterised in that the O-ring (130) is made of a biocompatible material sufficiently resistant to be able to compensate for the compressive forces.

## Patentansprüche

1. Vorrichtung zur Zwischenwirbelstabilisierung bestehend aus einem flexiblen Element, das zwischen mindestens zwei Wirbel mittels zweier jeweils jeden Wirbel zugeordneten Implantate in Stellung gebracht ist,
**dadurch gekennzeichnet,**
daß sie mindestens einen Dämpfer (1;10;10',10'',100) umfaßt, der geeignet ist, einerseits einer Dehnung und andererseits einer axialen Kompression zu widerstehen, wobei der Dämpfer (1;10;10',10'';100) die Form eines langgestreckten Körpers einnimmt, der mit einem Mittelteil (1a;12;12';12'';120) versehen ist, das mit zwei wulstigen Enden (1d,1e;11'a,11'b;110a,110b) die jeweils mit einem in den entsprechenden Wirbel (4,5) verankerten Implantat (2) zusammenarbeitet.

2. Vorrichtung nach Anspruch 1, dadurch gekennzeichnet, daß der Dämpfer (1;10;10';10'';100) starre Manschetten (7;70) umfaßt, die in der Nähe der Enden (1d,1e;11'a,11'b;110a,110b) angeordnet sind.

3. Vorrichtung nach Anspruch 1, dadurch gekennzeichnet, daß der Dämpfer (1,10,10',10'',100) aus einem elastischen bioverträglichen elastischen Material, wie einem Elastomer besteht.

4. Vorrichtung nach Anspruch 1, dadurch gekennzeichnet, daß der Dämpfer (1,10,10',10'',100) aus einem bioverträglichen Material mit zwei Komponenten besteht.

5. Vorrichtung nach Anspruch 1, dadurch gekennzeichnet, daß das Mittel (1a;12;12';12'';120) des Dämpfers (1;10;10';10'';100) einen Längsquerschnitt in elliptischer Form aufweist.

6. Vorrichtung nach Anspruch 1, dadurch gekennzeichnet, daß das Mittelteil (1a;12;12';12'';120) des Dämpfers (1;10;10';10'';100) asymmetrisch ist.

7. Vorrichtung nach Anspruch 1, dadurch gekennzeichnet, daß die Implantate (2), die die wulstartigen Enden (1d,1e;11'a,11'b;110a,110b) des Dämpfers (1;10;10';10'';100) aufnehmen, jeweils in Form einer Küvette (2c) realisiert sind, deren Boden (2e) ein konkaves Profil umfaßt, das eine für sein enges Zusammenwirken mit dem entsprechenden Ende des Dämpfers (1;10;10';10'';100) geeignete Form aufweist, wobei die Küvette Mittel (2d) zum Befestigen eines Einsatzes (3) aufweist, der zum Anlegen des Endes gegen den Boden (2e) und die Küvette (2c) des Implantats (2) dient.

8. Vorrichtung nach Anspruch 7, dadurch gekennzeichnet, daß die Küvette (2c) jedes Implantats (2) ein mit Gewinde versehenes Loch (2d) umfaßt, dessen Boden (2e) halbkugelförmig ist, wobei die Küvette mit einer Ausnehmung (2f) versehen ist, derart, daß sie seitlich nach außen mündet, und wobei die Befestigung des Endes des Dämpfers mittels eines mit Gewinde versehenen Einsatzes (3) mit halbkugelförmiger Grundfläche (3a) bewirkt wird, der in die Küvette geschraubt ist.

9. Vorrichtung nach Anspruch 8, dadurch gekennzeichnet, daß sie Mittelimplantate (2') aufweist, die zwei sich gegenüberstehende Ausnehmungen (2'f) für die Halterung der Enden von zwei angrenzenden (1') Dämpfern umfaßt, von denen mindestens ein Ende (1'd,1'e) halbkugelförmig ist.

10. Vorrichtung nach Anspruch 4, dadurch gekennzeichnet, daß eines der zwei Elemente des Dämpfers (10;10',10'',100) in Richtung der Ausdehnung und das zweite Element in Richtung der Kompression vorgespannt sind.

11. Vorrichtung nach Anspruch 1, dadurch gekennzeichnet, daß der Dämpfer (10') einen elastischen Kern (11'), der in Form einer Hantel ausgebildet ist, deren Stab elastisch gedehnt ist, sowie einen Körper (12') umfaßt, der durch die Elastizität des Kerns in Richtung der Kompression vorgespannt ist.

12. Vorrichtung nach Anspruch 1, dadurch gekennzeichnet, daß der Dämpfer (10'') in Form einer elastischen Hülle (11'') realisiert ist, die in axialer Richtung mit dem Ziel, sie zu verlängern, vorgedehnt ist und die einen einen Block (12'') aus elastischem Material einschließenden Hohlraum aufweist, den die Hülle durch Kompression vorspannt.

13. Vorrichtung nach Anspruch 1, dadurch gekennzeichnet, daß der Dämpfer (100) ein Element (110) und einen Körper (120) aufweist, die durch zwei Manschetten (70) verbunden sind, deren jeweiliges eines Ende in Richtung nach außen gekrümmt ist, um einen freien kreisförmigen Raum zu bilden, in dessen Innerem eine tonische Dichtung (130) eingefügt ist.

14. Vorrichtung nach Anspruch 13, dadurch gekennzeichnet, daß die tonische Dichtung (130) aus einem bioverträglichen Material hergestellt ist, das ausreichend widerstandsfähig ist, um die Kompressionsbelastungen zu kompensieren.
